(19) Europäisches Patentamt
European Patent Office
Office européen des brevets

(11) **EP 1 675 507 B1**

(12) **EUROPÄISCHE PATENTSCHRIFT**

(45) Veröffentlichungstag und Bekanntmachung des Hinweises auf die Patenterteilung:
**01.08.2007 Patentblatt 2007/31**

(21) Anmeldenummer: **04737418.6**

(22) Anmeldetag: **19.08.2004**

(51) Int Cl.:
*A61B 5/0225* (2006.01)    *A61B 5/022* (2006.01)

(86) Internationale Anmeldenummer:
**PCT/AT2004/000289**

(87) Internationale Veröffentlichungsnummer:
**WO 2005/037097 (28.04.2005 Gazette 2005/17)**

(54) **VORRICHTUNG UND VERFAHREN ZUR REGELUNG DES DRUCKES IN EINER AUFBLASBAREN MANSCHETTE EINES BLUTDRUCKMESSGERÄTES**

DEVICE AND METHOD FOR REGULATING THE PRESSURE IN AN INFLATABLE CUFF OF A BLOOD PRESSURE MANOMETER

DISPOSITIF ET PROCEDE POUR REGULER LA PRESSION DANS UN MANCHON GONFLABLE D'UN TENSIOMETRE

(84) Benannte Vertragsstaaten:
**AT BE BG CH CY CZ DE DK EE ES FI FR GB GR HU IE IT LI LU MC NL PL PT RO SE SI SK TR**

(30) Priorität: **21.10.2003 AT 16712003**

(43) Veröffentlichungstag der Anmeldung:
**05.07.2006 Patentblatt 2006/27**

(73) Patentinhaber: **CNSystems Medizintechnik GmbH 8020 Graz (AT)**

(72) Erfinder:
• **FORTIN, Jürgen**
  **A-8020 Graz (AT)**
• **GRÜLLENBERGER, Rupert**
  **A-8020 Graz (AT)**
• **HACKER, Alexander**
  **A-8141 Unterpremstätten (AT)**
• **SKRABAL, Falko**
  **A-8043 Graz (AT)**

(74) Vertreter: **Babeluk, Michael Patentanwalt, Mariahilfer Gürtel 39/17 1150 Wien (AT)**

(56) Entgegenhaltungen:
**EP-A- 1 155 659          US-A- 4 510 940**

• **WANG M ET AL: "Non-invasive continuous blood pressure monitoring by the unloading of vascular wall" IEEE ENGINEERING IN MEDICINE AND BIOLOGY SOCIETY 11TH ANNUAL INTERNATIONAL CONFERENCE, 9. November 1989 (1989-11-09), Seiten 1417-1418, XP010088431**

**Beschreibung**

Die Erfindung betrifft eine Vorrichtung und ein Verfahren zur Regelung des Druckes in zumindest einer aufblasbaren Manschette, vorzugsweise einer Fingermanschette, eines Blutdruckmessgerätes, welches eine plethysmographische Sensoreinrichtung aufweist, wobei ein plethysmographisches Signal PG und ein Manschettendrucksignal BP erfasst werden.

Mit der kontinuierliche Erfassung des Blutdruckes in einer Arterie auf unblutige (nicht-invasive) Weise beschäftigen sich Wissenschaftler und Forscher seit Jahren. Bereits 1942 stellte R. Wagner in München ein mechanisches System vor, das an der A. radialis mit der Hilfe der sog. "Vascular Unloading Technique" - dem Prinzip der entspannten Gefäßwand - den Druck in der Arterie aufzeichnen konnte (Wagner R. "Methodik und Ergebnisse fortlaufender Blutdruckschreibung am Menschen", Leipzig, Georg Thieme Verlag, 1942; Wagner R. et. al. "Vereinfachtes Verfahren zur fortlaufenden Aufschrift des Blutdruckes beim Menschen", Zschr. Biol. 112, 1960). Das 1973 von J. Penaz in Dresden vorgestellte Verfahren zur unblutigen Bestimmung des Blutdruckes (Digest of the 10th International Conference on Medical and Biological Engineering 1973 Dresden) wendet ebenfalls die Vascular Unloading Technique an. Diese ermöglichte erstmals eine - wenn auch nur kurze - kontinuierliche Registrierung des intraarteriellen Blutdruckes mit der Hilfe eines elektro-pneumatischen Regelkreises. Bei diesem Verfahren wird ein Finger durchleuchtet und durch eine Servoregelung wird ein Druck auf dem Finger so angebracht, dass der durch die Durchleuchtung registrierte, ursprünglich pulsatile Fluss konstant gehalten wird.

Dieses Verfahren stellt im Prinzip die folgende Regelschleife dar: Eine Extremität des menschlichen Körpers in der sich eine Arterie befindet, wie zum Beispiel Finger, Handwurzel oder die Schläfe, wird mit einer Lichtquelle durchleuchtet. Das Licht, das diese Extremität durchströmt (z.B. Finger) oder am in der Extremität liegenden Knochen reflektiert wird (z.B. Handwurzel, Schläfe), wird mit einem geeigneten Lichtdetektor registriert und ist ein inverses Maß für das Blutvolumen in der Extremität (plethysmographisches Signal PG). Je mehr Blut sich in der Extremität befindet, desto mehr Licht wird absorbiert und desto kleiner ist das plethysmographische Signal PG. Das PG wird mittels Differenzverstärker von seinem Mittelwert bereinigt und einem Regler zugeführt. Bei Penaz hat dieser Regler eine sogenannte Proportional - Integral - Differential (PID) Charakteristik. Der vom PID-Regler gebildete Stellwert wird verstärkt, zu einem konstanten Arbeitspunkt (Setpoint SP) addiert und einem Servo- oder Proportionalventil zugeführt, das einen Druck in einer Manschette erzeugt, die auf die vom Licht durchleuchtete Extremität wirkt. Die Regelbedingung legt fest, dass das plethysmographische Signal PG über die Zeit durch den anliegenden Druck konstant gehalten wird. Wenn während der Systole das Herz mehr Blut in die Extremität gepumpt wird und das PG die Tendenz zeigt kleiner zu werden, so erhöht der PID-Regler seinen Stellwert und der Druck in der anliegenden Manschette steigt so lange, bis dass das überschüssige Blut herausgedrückt wird und das PG wieder ausgeglichen wird. Umgekehrt, wenn während der Diastole weniger Blut in die Extremität fließt, weil sich das Herz in der Füllphase befindet und daher das PG steigen würde, senkt der PID-Regler seinen Stellwert und vermindert dadurch den Druck am Finger. Das plethysmographische Signal PG bleibt somit konstant. Durch diese Regelbedingung, dass das PG und somit das Blutvolumen in der Extremität über die Zeit konstant bleibt, ist der Druckunterschied (der sogenannte transmurale Druck) zwischen dem intraarteriellen Druck und dem außen anliegenden Druck gleich Null. Somit entspricht der außen anliegende Druck, nämlich der Manschettendruck BP, dem intraarteriellen Druck in der Extremität. Dieser kann mittels eines Drucksensors bzw. Manometers somit indirekt gemessen werden.

Bei der oben angeführte Beschreibung des Penaz-Prinzips befindet sich der Regelkreis in der sogenannten "Closed Loop" Operation. Die Regelschleife kann aber auch geöffnet werden (Open Loop), wobei der Stellwert des PID-Reglers nicht mit dem Arbeitspunkt (Setpoint SP) addiert wird. Der Druck in der Manschette ist deshalb vom plethysmographischen Signal PG unabhängig und wird vom Setpoint SP vorgegeben. In dieser Betriebsart wird der optimale SP an der Extremität ermittelt. Dieser SP entspricht laut Penaz dem mittleren arteriellen Blutdruck in der Extremität und ist dadurch gekennzeichnet, dass die Pulsationen des PG am größten sind.

Diese photo-plethysmographische Methode wurde in einigen weiteren Verfahren und Vorrichtungen zur Blutdruckmessung aufgegriffen. Die EP 0 537 383 A1 zeigt eine aufblasbare Fingermanschette für die nicht-invasive kontinuierliche Blutdrucküberprüfung. Der aufblasbare zylindrische Raum ist pneumatisch mit einer Fluidquelle verbunden. Eine Infrarotlichtquelle und ein Detektor sind beidseitig des Fingers innerhalb eines festen Zylinders positioniert. Weiters ist ein Ventil zum Füllen des Zylinders mit Gas vorgesehen. Es sind elektrische Kabel für die Infrarotlichtquelle und den Detektor durch den Zylinder hindurchgeführt. Die US 4,510,940 A und die US 4,539,997 A zeigen Vorrichtungen und Verfahren zur kontinuierlichen nicht-invasiven Messung des Blutdruckes. Es sind eine fluidgefüllte Manschette, eine Lichtquelle, ein Lichtdetektor und ein Differenzdruckverstärker vorgesehen. Ähnliche Vorrichtungen zur Blutdruckmessung sind aus der US 4,406,289 A bekannt. Dokument US 4,510,940A offenbart ein Verfahren bzw. eine Vorrichtung zur Druckregelung in einer aufblasbaren Manschette eines Blutdruckmessgeräts mit plethysmographischer Sensoreinrichtung. Dabei sind für das Manschettendrucksignal und für das plethysmographische Signal je ein separater Regelkreis vorgesehen. Die US 4,510,940A ist der nächstliegende Stand der Technik.

Aus der WO 00/59369, die ein kontinuierliches, nicht-invasives Blutdruckmessgerät zum Gegenstand hat, ist eine Verbesserungen des Proportionalventils bzw. des Druckerzeugungssystem bekannt, sowie unterschiedliche Ausführungen der Druckmanschetten für verschiedenen Extremitäten dar.

Alle bekannten Verfahren und Vorrichtungen haben - obwohl sie zum Teil wesentliche Verbesserungen bezüglich Manschette, Proportionalventil, Ermittlung des Arbeitspunktes SP, usw. betreffen - mit dem ursprünglichen Messprinzip von Penaz eines gemeinsam: Einen relativ einfachen Regelkreis in der "Closed Loop" Betriebsart mit einem Regler, beispielsweise einem PID-Regler. Der von Penaz beschriebene Regelkreis stellt eine Herausforderung an die Regeltechnik dar. Folgende unabhängige Systeme mit jeweils spezifischen Störgrößen sind Teil der Regelstrecke:

- Druckerzeugung mit Druckquelle (Pumpe) und Proportionalventil - Pumpendruck und Leckage des Ventils können sich ändern.

- Druckkammer, Manschette sowie Druckübertragung auf arterielles Gefäßsystem durch das Gewebe der Extremität.

- Pulsatile Blutflussschwankungen hervorgerufen durch die Herzaktion - die eigentliche Störgröße, die durch den Manschettendruck gemäß Penaz-Prinzip ausgeglichen werden soll.

- Das arterielle Blutgefäß ist, wenn als Extremität der Finger verwendet wird, ein sogenanntes Widerstandsgefäß. Das bedeutet, dass der Durchmesser der Arterie - und somit auch das Blutvolumen - durch die glatte Gefäßmuskulatur über das vegetative Nervensystem vergrößert (Vasodilation) und verkleinert (Vasoconstriktion) werden kann.

- Lichterzeugungs- und Detektionssystem. Störgrößen sind hier neben Bauteiltoleranzen vor allem der Einfluss des Umbebungslichtes auf das plethysmographische Signal PG.

- Mittelwertbereinigung des PG.

- Weitere Störgrößen auf Grund von Bauteilschwankungen, elektronischen und mechanischen Einflüssen.

Diese Faktoren machen es fast unmöglich, selbst bei optimal detektierten Arbeitspunkt SP in der Open Loop Betriebsart, eine kontinuierliche Blutdruckmessung nach dem Penaz Prinzip über eine längere Dauer durchzuführen.

In der US 4,510,940 A wurde versucht diese Nachteile zu beheben. Es wird ein Verfahren zur langfristigen Blutdruckmessung beschrieben, bei welchem die Open Loop Betriebsart periodisch unterbrochen wird und in der Closed Loop Betriebsart der SP neu detektiert wird. Diese Methode stellt daher nur einen Kompromiss dar und hat den Nachteil, dass Blutdruckschwankungen während der periodischen Suche nach dem optimalen SP nicht detektiert werden.

Aufgabe der vorliegenden Erfindung ist es, ausgehend von den eingangs beschriebenen Verfahren und Vorrichtungen ein verbessertes Regelverfahren bzw. eine entsprechende Vorrichtung zur Durchführung des Verfahrens für ein Blutdruckmessverfahren vorzuschlagen, bei welchem ein plethysmographisches Signal PG und ein Manschettendrucksignal BP erfasst werden. Dabei soll insbesondere eine langfristige, indirekte Messung des kontinuierlichen Blutdruckes gewährleistet sein.

Diese Aufgabe wird erfindungsgemäß dadurch gelöst,

a) dass in einem ersten, inneren Regelkreis das Manschettendrucksignal BP als Regelgröße verwendet und als erstes Eingangssignal einem Differenzverstärker zugeführt wird,

b) dass in einem zweiten, äußeren Regelkreis das plethysmographische Signal PG um seinen Mittelwert **PG** bereinigt einer Regeleinrichtung, vorzugsweise einem PID-Regler, zugeführt zu einem Arbeitspunktsignal SP addiert und ein Sollwertsignal SW generiert wird, welches als zweites Eingangssignal dem Differenzverstärker zugeführt wird, sowie

c) dass mit dem Ausgangssignal AS des Differenzverstärkers zumindest ein mit einer Druckquelle verbundenes Ventil, vorzugsweise ein Proportionalventil, zur Regelung des Druckes in der Manschette angesteuert wird.

Eine Vorrichtung zur Regelung des Druckes in zumindest einer aufblasbaren Manschette, vorzugsweise einer Fingermanschette, eines Blutdruckmessgerätes, welches eine plethysmographische Sensoreinrichtung zur Erfassung eines plethysmographischen Signals PG und einen Drucksensor zur Erfassung eines Manschettendrucksignals BP aufweist, zeichnet sich dadurch aus, dass zwei auf einen Differenzverstärker wirkende Regelkreise vorgesehen sind, wobei der erste, innere Regelkreis das Manschettendrucksignals BP als erste Regelgröße verwendet und der zweite, äußere Regelkreis eine Regeleinrichtung, vorzugsweise einen PID-Regler, aufweist, welche bzw. welcher aus dem plethysmographischen Signal PG einen Sollwert SW als zweite Regelgröße generiert, sowie dass der Differenzverstärker ausgangsseitig zumindest ein mit einer Druckquelle verbundenes Ventil, vorzugsweise ein Proportionalventil, zur Regelung des Druckes in der Manschette steuert. Der zweite Regelkreis weist einen an sich bekannten Differenzverstärker auf, welcher das plethysmographische Signal PG von seinem Mittelwert $\overline{PG}$ subtrahiert, sowie eine Summationseinheit (13), welche ein Arbeitspunktsignal SP addiert.

Die vorliegende Erfindung beschreibt nun ein neuartiges Regelungsverfahren, das eine langfristige indirekte Messung des kontinuierlichen Blutdruckes gewährleistet. Das Regelungssystem kann sowohl als elektronische Schaltung aufgebaut werden als auch weitgehend in einem Rechner mit Programm- und Datenspeicher umgesetzt werden. Periphere Regelkreise kann man vorzugsweise in einem Rechner als Programmlogik aufbauen, schnellere innere Regelkreise, die auch die Treiber für das Druckerzeugungssystem oder das Lichterzeugungs- und Lichtdetektionssystem beinhalten, werden vorzugsweise als elektronische Schaltung aufgebaut. Eine genaue Abgrenzung zwischen Programmlogik und elektronischer Schaltung ist erfindungstechnisch nicht notwendig.

Das grundlegende Prinzip des gegenständlichen Regelverfahrens besteht darin, für genau bestimmte zeitliche Eigenschaften und Parameter der Regelstrecke (schneller Druckaufbau und Abbau, Ausgleich des transmuralen Druckes über einen einzelnen Herzzyklus, mittelfristige Veränderungen, langfristige Drifts) eigene Regelkreise vorzusehen, die vorzugsweise konzentrisch angeordnet sind. Konzentrisch bedeutet in diesem Fall, dass der innere Regelkreis für eine bestimmte zeitliche Eigenschaft bzw. Parameter der Regelstrecke verantwortlich ist und dem unmittelbar äußeren Regelkreis idealisierte Bedingungen für diese jeweilige zeitliche Eigenschaft zur Verfügung stellt. Dieser unmittelbar äußere Regelkreis kann nun wiederum ein innerer Regelkreis für den nächsten äußeren Regelkreis sein. Vorzugsweise sind die weiter innen liegenden Regelkreise für die schnellen Regelvorgänge verantwortlich, die weiter außen liegenden Regelkreise sind für die langfristige Stabilität des Regelsystems verantwortlich. Weiters können auch für bestimmte spezifische Größen (wie z.B. Druck in der Manschette, Lichtdetektionssystem, Mittelwertbereinigung oder andere) eigene Regelkreise mit den für die jeweilige Störgröße optimierten Regelparametern vorhanden sein. Diese Regelkreise müssen nicht unbedingt im vorhin beschriebenen Sinne konzentrisch angeordnet sein.

Die Erfindung wird im Folgenden anhand von Zeichnungen näher erläutert. Es zeigen:

Fig. 1 eine erfindungsgemäße Vorrichtung zur Regelung des Druckes in einer aufblasbaren Manschette eines Blutdruckmessgerätes mit zwei Regelkreisen in einer teilweise schematischen Darstellung,

Fig. 2 eine erweiterte Ausführungsvariante der Vorrichtung nach Fig. 1 mit zusätzlichen Regelkreisen,

Fig. 3 eine Variante der Erfindung mit separatem Ein- und Auslassventil für die aufblasbare Manschette, sowie

Fig. 4 Schaltungsdetails einer weiteren Ausführungsvariante der Regelvorrichtung.

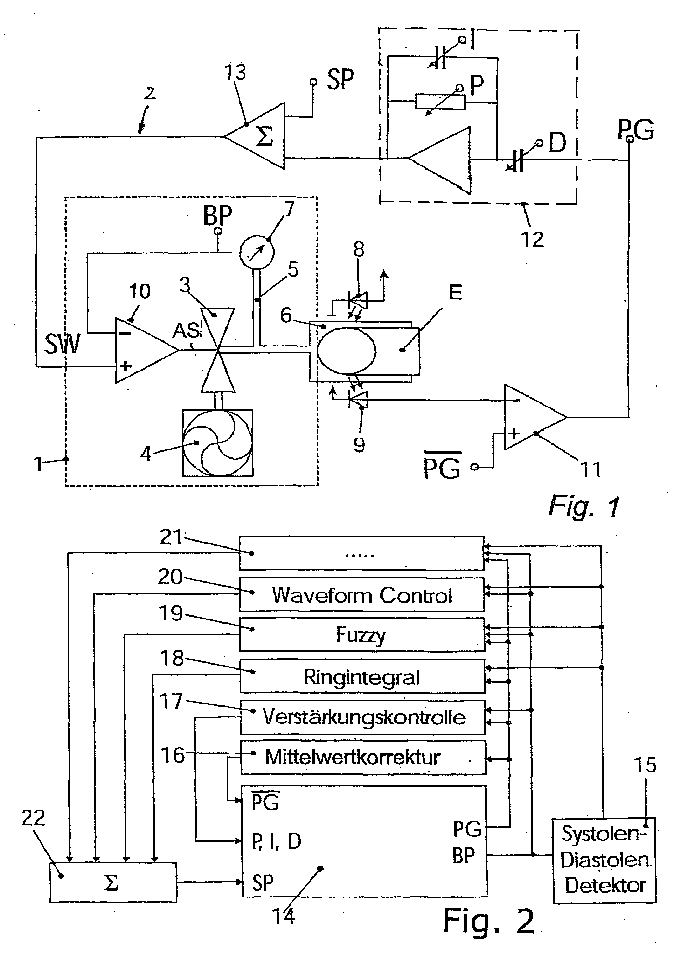

Fig. 1 zeigt eine Vorrichtung zur Regelung des Druckes in einer aufblasbaren Fingermanschette 6, eines nicht weiter dargestellten Blutdruckmessgerätes. Die Regelvorrichtung besteht aus einem ersten, inneren Regelkreis 1, dem ein Sollwertsignal SW von einem zweiten, äußeren Regelkreis 2 vorgegeben wird. Der innere Regelkreis 1 weist einen Differenzverstärker 10 auf (vorzugsweise einen Operationsverstärker), ein Proportionalventil 3, dem ein Druck aus einer Druckquelle, z.B. einer Pumpe 4, zugeführt wird, einer Druckkammer 5, welche an die Manschette 6 angeschlossen ist und einen Drucksensor 7, der den in der Druckkammer 5 bzw. Manschette 6 erzeugten Druck in ein, dem Manschettendruck proportionales, elektrisches Signal BP umwandelt. Dieses elektrische Signal BP, welches dem intraarteriellen Druckverlauf in der Extremität E entspricht, wird dem Differenzverstärker 10 zugeführt, womit die erste, innere Regelschleife 1 geschlossen ist. Der Differenzverstärker 10 stellt seine Ausgangsspannung AS so ein, dass die Spannung zwischen seinem +Eingang und -Eingang gleich Null wird. Der Differenzverstärker 10 verstellt somit den Druck in der Manschette 6 über das Proportionalventil 3 derartig, dass die Spannung, die der Drucksensor 7 erzeugt gleich dem Sollwert SW ist. Der zweite, äußere Regelkreis 2 gibt einen Sollwert SW vor, der dem tatsächlichen Druck an der Extremität E (z.B. Finger) entspricht, welcher für die Konstanthaltung des plethysmographischen Signals PG der Plethysmographischen Sensoreinrichtung 8, 9 notwendig ist. Der äußere Regelkreis 2 ist nun nicht mehr für die spezifischen Eigenschaften des Druckerzeugungssystems, bestehend aus dem Proportionalventil 3, der Pumpe 4, der Druckkammer 5, der Manschette 6 sowie dem Drucksensor bzw. Manometer 7, verantwortlich und besteht im Wesentlichen aus der plethysmographischen Sensoreinrichtung nämlich einer Lichtquelle 8 (vorzugsweise LED) und einem Lichtdetektor 9 (vorzugsweise Photodiode), die das Blutvolumen in der Extremität E in der bekannten Weise bestimmen sowie einem Differenzverstärker 11, der das Signal PG von seinem Mittelwert $\overline{PG}$ subtrahiert und einem Regler 12, bei dem die Regelparameter Proportionalverstärkung P, Integralverstärkung I und/oder Differentialverstärkung D eingestellt werden können. Der äußere Regelkreis 2 wird über einen Summationseinheit 13 geschlossen, die das Regelungssignal zum vorgebbaren Arbeitspunktsignal SP addiert und somit den Sollwert SW für den inneren Regelkreis 1 vorgibt.

Die in Fig. 1 beschriebene, einfache Darstellung des erfindungsgemäßen Regelverfahrens hat gegenüber den eingangs beschriebenen Verfahren den Vorteil, dass der innere Regelkreis 1 für schnelle Druckänderungen optimiert ist, der äußere Regelkreis 2 ist nun ausschließlich für den Ausgleich des plethysmographischen Signals PG verantwortlich. Die jeweiligen Regelparameter können so für die jeweilige Aufgabe optimiert werden. Ein weiterer Unterschied, auf den noch später näher eingegangen wird, ist die Tatsache, dass es hier keine eindeutige Abgrenzung zwischen Open Loop und Closed Loop Betriebsart gibt. Die Open Loop Betriebsart ist beim erfindungsgemäßen Verfahren dann gegeben, wenn die Schleifenverstärkungen P, I und D des PID-Reglers 12 auf Null gesetzt werden. Alle anderen Einstellung dieser Parameter schließen den Regelkreis 2.

Falls auch langfristige Änderungen des Systems ausgeglichen werden sollen, können weitere Regelkreise, die jeweils für eine andere spezifische Aufgabe verantwortlich sind, hinzugefügt werden. Regelkreise, die während der Blutdruckaufzeichnung (Closed Loop Betriebsart) bestimmte zeitliche Veränderungen ausgleichen (langsame mittel- bis langfristige Drifts, aber auch schnellere Druckänderungen) werden vorzugsweise konzentrisch im eingangs beschriebenen Sinne angebracht. Regelkreise bzw. Verfahren, die bestimmte Anfangswerte (Anfangswerte SP, P, I und D, Einstellungen des Lichterfassungssystems, Mittelwertbereinigung des PG, usw.) für die eigentliche Messung festlegen, müssen nicht unbedingt konzentrisch angelegt sein.

Fig. 2 zeigt eine mögliche Erweiterung der beiden Regelkreise 1 und 2 aus der Grundschaltung gemäß Fig. 1. Die hier angeführten Regelkreise 16 bis 21 müssen nicht alle eingesetzt sein und auch die Reihenfolge kann verändert werden. Es soll mit der schematischen Darstellung gemäß Fig. 2 das Grundprinzip der konzentrischen Regelkreise verdeutlicht werden, sowie die Neuerungen gegenüber dem Stand der Technik dargestellt werden. Vorzugsweise sollen aber Regelkreise, die öfter in das Gesamtsystem eingreifen und deswegen für schnellere Änderungen zuständig sind, weiter innen liegen.

Die Regelschaltung gemäß Fig. 1 mit den Regelkreisen 1 und 2 ist in Fig. 2 schematisch als zentrale Regeleinrichtung 14 dargestellt und hat die Eingangsparameter SP, $\overline{PG}$, P, I und D sowie die Ausgangsparameter BP und PG. Um die zentrale Regeleinrichtung 14 können nun weitere konzentrische Regler eingesetzt werden, ohne dass die Messung unterbrochen werden muss, um neue Anfangsbedingungen in einer Open Loop Betriebsart zu bestimmen, wie dies gemäß US 4,510,940 A der Fall ist. In der eingangs zitierten US 4,539,997 A wird zwar rein formal auch von einer ersten und zweiten Regelschleife gesprochen, im Unterschied zur Erfindung ist hier aber die bekannte Closed Loop Regelschleife mit einem PID-Regler sowie eine Open Loop Regelschleife ohne Regler gemeint

Erfindungsgemäß wird das Manschettendrucksignal BP einem Systolen-/Diastolendetektor zugeführt, dessen Ausgangssignal als Regelgröße in zumindest einem der weiter unten beschriebenen Regelkreise drei bis acht verwendet wird. Der für bestimmte Regelkreise notwendige Zeitpunkt der Systolen bzw. Diastolen des Blutdruckes wird durch diesen Detektor zur Verfügung gestellt.

Beispielsweise wird erfindungsgemäß in einem dritten Regelkreis (Mittelwertkorrektur 16) der Mittelwert $\overline{PG}$ aus dem plethysmographischen Signal PG bestimmt und als Eingangsgröße des zweiten Regelkreises laufend korrigiert. Dieser Regler ermittelt den Mittelwert des PG und regelt diesen gegebenenfalls am $\overline{PG}$ - Eingang des zweiten Regelkreises nach.

In einer Weiterbildung der Erfindung werden in einem vierten Regelkreis (Verstärkungskontrolle 17) die Verstärkungsparameter P, I und/oder D anhand des plethysmographischen Signals PG und des Manschettendrucksignal BP optimiert und als Eingangsgrößen des PID-Reglers 12 laufend korrigiert Dieser Regelkreis dient der Kontrolle und ggf. der Korrektur der Schleifenverstärkungen P, I und D des zweiten Regelkreises. Dazu wird das Verhältnis zwischen Manschettendrucksignal BP und plethysmographischem Signal PG ständig kontrolliert und optimiert.

In einer vorteilhaften Ausführungsvariante der Erfindung wird in einem fünften Regelkreis 18 das Arbeitspunktsignal SP in Abhängigkeit des Integrals des plethysmographischen Signals PG nachgeregelt. Hier wird ein Integralwert des PG zwischen zwei Diastolen berechnet. Trotz ständiger Ausregelung ist natürlich eine kleines PG-Signal als Regelabweichung vorhanden und somit kann auch das Integral dieses Signals berechnet werden. Da die Regelbedingung des gesamten Systems verlangt, dass das plethysmographische Signal PG durch den anliegenden Druck konstant gehalten wird, muss auch das Integral des PG über die Zeit bzw. über einen Herzzyklus konstant bleiben. Ist dies nicht der Fall, greift der Regelkreis 18 in das System ein und verändert den anliegenden Druck durch Veränderung des Arbeitspunktes SP.

Gemäß einer erfinderischen Weiterbildung wird in einem sechsten Regelkreis (Fuzzy-Regelkreis 19) das Arbeitspunktsignal SP auf der Basis abgeleiteter Größen, wie z.B. Amplitude, Mittelwert, Signalform etc., des plethysmographischen Signals PG und des Manschettendrucksignals BP mit Hilfe eines Fuzzy-Logik-Ansatzes nachgeregelt. Der Fuzzy-Regler 19 vergleicht die jeweils neuen, durch den Systolen - Diastolendetektor 15 getrennten Herzzyklen mit vorhergegangenen Herzzyklen. Dabei werden beide Signale BP und PG kontrolliert. Gemäß der Fuzzy-Logik können nun zum Beispiel folgende Fuzzy Heurisken formuliert werden:

- BP bzw. PG ist (stark) größer/kleiner geworden, deswegen wird SP rauf/runter geregelt

- Verhältnis von mittleren Druck zur Druckamplitude ist größer/kleiner geworden, deswegen wird SP rauf/ runter geregelt

- Verhältnis von mittleren Druck zum diastolischen Druck ist größer/kleiner geworden, deswegen wird SP rauf/runter geregelt

- usw.

Erfindungsgemäß wird in einem siebenten Regelkreis 20 das Arbeitspunktsignal SP in Abhängigkeit der Pulswellenform des Manschettendrucksignals BP nachgeregelt. Der Regler 20 "Waveform Control" vergleicht ebenfalls die jeweils neuen, durch den Systolen - Diastolendetektor 15 getrennten Herzzyklen mit vorhergegangenen, wobei die Form des Manschettendrucksignals BP kontrolliert und mit der Pulswellenform vorhergegangener Herzzyklen verglichen wird. Die Form der Pulswelle ist bekannter Weise von Patienten zu Patienten verschieden - jeder Patient hat quasi seine eigene Pulswellenform, wie er auch seinen eigenen Fingerabdruck besitzt. Die Form der Pulswelle ist abhängig vom Zustand der großen und kleinen Gefäße und ändert sich über die Jahre - nicht aber während einer Blutdruckmessung. Diese Eigenschaft kann man sich auch für die Regelung des Manschettendruckes zu eigen machen. Ändert sich die Pulswellenform über die Zeit, so ist wahrscheinlich eine physiologische Vasokonstriktion oder Vasodilation aufgetreten und der Arbeitspunkt bzw das Arbeitspunktsignal SP muss nachgeregelt werden.

Schließlich kann in einem achten Regelkreis 21 das Arbeitspunktsignal SP mit Hilfe neuronaler Netze, autoregressiver Modelle oder selbstlernender Modelle nachgeregelt werden

Die Summationseinrichtung 22 (Fig. 2) addiert die von den jeweiligen Regelkreisen 16 bis 17 vorgeschlagenen Änderungen des Arbeitspunktes SP und stellt den an der Extremität E anliegenden Druck über den SP-Eingang des zweiten Regelkreises 2 (Fig. 1) nach.

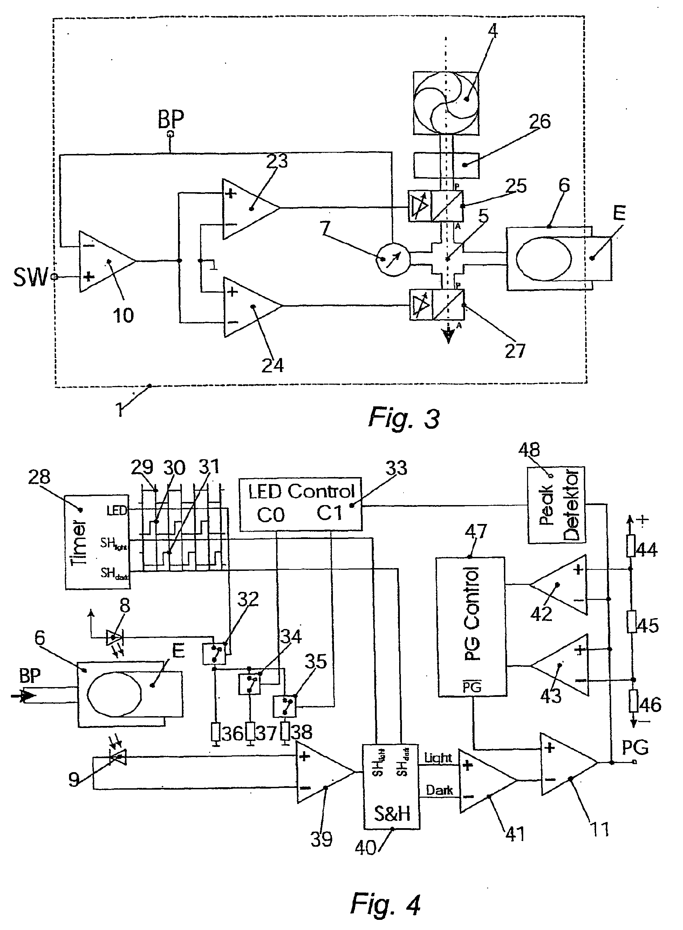

Fig. 3 zeigt eine erfindungsgemäße Ausführung, bei welcher der Differenzverstärker 10 ausgangsseitig über eine nicht-invertierende Verstärkereinheit 23 ein mit der Druckquelle 4 verbundenes Einlassventil 25 und über eine invertierende Verstärkereinheit 24 ein Auslassventil 27 steuert, welche vorzugsweise als Proportionalventile ausgeführt sind und mit der aufblasbaren Manschette 6 in Druckverbindung stehen. Anstatt eines Proportionalventils 3 (wie in Fig. 1) werden hier zwei separate Ventile - eines für den Druckaufbau und eine für den Druckabbau - verwendet. Die Vorteile einer solchen Anordnung, allerdings ohne Anwendung mehrerer Regelkreise, ist in der eingangs zitierten WO 00/59369 A2 beschrieben.

Der in Fig. 3 dargestellte, alternative Regelkreis 1, dem ein Sollwert SW vorgegeben wird, besteht aus dem Differenzverstärker 10, (vorzugsweise ein Operationsverstärker), welcher als Regler dient. Die Ausgangsspannung des Differenzverstärkers 10 treibt eine nicht-invertierende Verstärkereinheit 23 sowie eine invertierende Verstärkereinheit 24. Die absolute Verstärkung beider Einheiten ist gleich, so dass die Ausgangspannung der einen Einheit genau gegengleich der Spannung der anderen Einheit ist.

$$U_1 = - U_2.$$

Die Verstärkereinheit 23 steuert ein proportionales Einlassventil 25, das auf den einen Seite über ein Druckausgleichgefäß 26 mit der Pumpe 4 verbunden ist. Dieses Einlassventil 25 steuert den Einlassdruck in eine Druckkammer 5, die mit der Manschette 6 in Druckverbindung steht. Die Verstärkereinheit 24 steuert ein proportionales Auslassventil 27, das auf den einen Seite mit der Druckkammer 5 verbunden ist. Dieses Auslassventil 27 steuert den Auslassdruck der Druckkammer 5 gegenüber dem normalen atmosphärischen Druck. Steigt die Ausgangsspannung des Differenzverstärkers 10, so steigt die Ausgangsspannung der ' nicht-invertierende Verstärkereinheit 23 und die Ausgangsspannung der invertierenden Verstärkereinheit 24 sinkt im gleichen Maße. Somit wird das Einlassventil 25 geöffnet und das Auslassventil 27 im gleichen Maße geschlossen. Der Druck in der Manschette 6 steigt rasch an. Sinkt hingegen die Ausgangsspannung des Differenzverstärkers 10, so passiert genau das Gegenteil. Das Auslassventil 27 wird über die invertierende Verstärkereinheit 24 geöffnet und im gleichen Maße wird das Einlassventil 25 über die nicht-invertierende Verstärkereinheit 23 geschlossen, wodurch der Druck in Druckkammer 5 und in der Manschette 6 sinkt. Ein Drucksensor bzw. Manometer 7 wandelt den in der Druckkammer 5 erzeugten Druck in das, dem Druck proportionale, Manschettendrucksignal BP um, welches. dem Differenzverstärker 10 zugeführt wird, womit die erste Regelschleife geschlossen wird. Idealerweise stellt der Differenzverstärker 10 seine Ausgangspannung so ein, dass die Spannung zwischen seinem +Eingang und -Eingang gleich Null wird. Der Differenzverstärker 10 verstellt über die Verstärkereinheiten 23 und 24 das Einlassventil 25 und das Auslassventil 27 derartig, dass die Spannung, die der Drucksensor 7 erzeugt gleich dem Sollwert SW ist.

Die in Fig. 3 angeführte Schaltung funktioniert in vorteilhafter Weise auch mit nicht-linearen Ventilen 25 und 27 - mehr noch, sie funktioniert auch mit schnellen digitalen Auf-Zu-Schaltventilen. Erfindungsgemäß kann der Differenzverstärker 10 als Komparator ausgeführt sein, welcher zumindest ein digitales Schaltventil zur Regelung des Druckes in der Manschette 6 steuert. Der Komparator entspricht dabei einem Operationsverstärker mit maximaler Verstärkung (ohne Verstärkungsrückkoppelung). Der Komparator 10 vergleicht SW und BP. Ist BP kleiner als SW, so ist die Ausgangspannung annähernd gleich der positiven Betriebsspannung und über die Verstärkereinheiten 23 wird das Einlassventil 25 komplett geöffnet und über die Verstärkereinheit 24 das Auslassventil 27 komplett geschlossen. Der in der Druckkammer 5 erzeugte Druck steigt bis BP größer ist als SW. Dann ist die Ausgangsspannung des Differenzverstärkers (Komparator) 10 zirka gleich der negativen Betriebsspannung und somit das Einlassventil 25 komplett geschlossen sowie das Auslassventil 27 komplett geöffnet. Der in der Druckkammer 5 erzeugte Druck sinkt. Sind SW und BP annähernd gleich, dann entsteht am Ausgang des Differenzverstärkers (Komparator) 10 ein Rechtecksignal mit 50% Tastverhältnis. Die Information über Druckanstieg bzw. Abfall liegt also im Tastverhältnis des am Ausgang des Differenzverstärkers (Komparator) 10 erzeugten Rechecksignals. Voraussetzung für das Funktionieren sind hinreichend schnelle Schaltventile (vorzugsweise Piezoventile) die weitaus schneller reagieren als es der Trägheit der Druckänderung in der Manschette 6 entspricht.

Die anhand der Fig. 1 bis Fig. 3 dargestellten Regelkreise arbeiten alle während der kontinuierlichen Blutdruckmessung (Closed Loop Betriebsart). Für den einwandfreien Betrieb müssen - wie für die meisten Regelkreise - klar definierte Startwerte bestimmt werden. Diese Startwerte werden vorzugsweise vor dem Beginn der eigentlichen Messung bestimmt. Ob dies in der Open Loop oder Closed Loop Betriebsart geschieht ist dabei unerheblich.

Anders als bei den eingangs zum Stand der Technik erwähnten Verfahren und Vorrichtungen, ist bei der Erfindung das Einstellen eines optimalen plethysmographischen Signals PG von Vorteil. Erfindungsgemäß weist daher die plethysmographische Sensoreinrichtung 8, 9 eine Einrichtung 28, 40 41 zur Bereinigung des plethysmographischen Signals PG von Fehllicht, insbesondere von Umgebungslicht auf, wobei weiters eine Einrichtung 33 bis 38 zur Steuerung der Spannung bzw. des Stroms der Lichtquelle 8 der plethysmographischen Sensoreinrichtung vorgesehen ist.

In Fig. 4 wird ein möglicher Regelkreis dargestellt, der ein optimales PG-Signal liefert. Der Teil des Reglers, der das Umgebungslicht bereinigt, ist ein konzentrischer Regelkreis, der Teil, der den optimalen LED-Strom (Lichtquelle 8) einstellt hingegen nicht, weil dieser Teil einen Startwert festlegt.

Zur Ansteuerung der Lichtquelle 8, einer LED, dient ein Timer 28, welcher drei zeitsynchrone Rechtecksignale erzeugt. Das Signal "LED" 29 dient zur pulsatilen Ansteuerung der LED 8. Wenn das Signal "LED" 29 auf HIGH-Level steht, wird die LED 8 eingeschaltet. Das in Fig. 4 dargestellte 50%ige Tastverhältnis ist nicht unbedingt notwendig, es können auch andere Verhältnisse eingesetzt werden. Weiters wird vom Timer 28 auch das Signal "Sh$_{light}$" 30 erzeugt, welches einen HIGH-Level unmittelbar vor dem Ausschalten der LED 8 aufweist. Das weiters vom Timer 28 erzeugte Signal "Sh$_{dark}$" 31 hat einen HIGH-Level unmittelbar vor dem Einschalten der LED 8.

Das Signal 29 schaltet die LED 8 mit dem Schalter 32 ein. Mit der Einheit LED Control 33 kann die Stromstärke durch die LED 8 und somit die Lichtintensität verändert werden. Mit den Schaltern 34 und 35 können zum Strombegrenzungswiderstand 36 parallel liegende Widerstände 37 und 38 dazugeschaltet werden und somit der Gesamtstrom durch die LED 8 erhöht werden.

Das Licht, das die Extremität E durchströmt, wird von einer Photodiode 9 detektiert und von einem Verstärker 39 verstärkt. Dieses detektierte Lichtsignal schwankt nun pulsatil mit dem Ein- und Ausschalten der LED 8. Aber auch wenn die LED 8 nicht leuchtet wird ein schwaches Lichtsignal empfangen, weil auch das Umgebungslicht die Extremität E durchströmt und ein Signal auf der Photodiode 9 erzeugt. Um Einschwing- und Abklingvorgänge durch das Ein- und Ausschalten der LED 8 zu vermeiden, werden nun die Zeitpunkte unmittelbar vor dem Einschalten bzw. dem Ausschalten der LED 8 betrachtet. Unmittelbar vor dem Einschalten der LED 8 - also noch bei dunkler LED 8 - ist das Signal, das in der Photodiode 9 erzeugt wird rein vom Umgebungslicht abhängig. Umgekehrt - unmittelbar vor dem Ausschalten der LED 8 - also bei heller LED 8 - ist das Signal, das in der Photodiode 9 erzeugt wird vom Licht der LED 8 und dem Umgebungslicht abhängig. Diese Zeitpunkte sind durch den Timer 28 und seinen Signalen "Sh$_{light}$" 30 und "Sh$_{dark}$" 31 definiert. Das verstärkte Lichtsignal von Photodiode 9 wird nun auf ein Sample & Hold-Glied 40 gelegt und durch die Signale "Sh$_{light}$" 30 und "Sh$_{dark}$" 31 demoduliert. An den Ausgängen des Sample & Hold-Glied 40 entstehen die Signale Light und Dark. Subtrahiert man die beiden Signale in einem Differenzverstärker 41, so entsteht ein Lichtsignal, das nur von der Leuchtstärke der LED 8 abhängig ist und somit vom Umgebungslicht bereinigt worden ist.

Das erzeugte Lichtsignal besteht aus einem überwiegenden Teil aus einem Gleichanteil und zu einem kleineren Teil aus dem gewünschten plethysmographischen Signal PG, das der pulsatilen Veränderung des Blutvolumens aufgrund der Herzaktionen entspricht. Dieser Gleichanteil bzw. Mittelwert des PG-Signals $\overline{PG}$ ist für die eigentliche Messung des Blutdruckes nicht relevant und störend und das Lichtsignal muss daher vom Mittelwert $\overline{PG}$ bereinigt werden. Dieser $\overline{PG}$ ist aber von der jeweiligen Extremität E abhängig und unterscheidet sich stark von Patienten zu Patienten. Die Regelvorrichtung weist daher erfindungsgemäß eine Einrichtung 42 bis 47 zur Berechnung eines Startwertes für den Mittelwert $\overline{PG}$ des plethysmographischen Signals auf. Die Mittelwertskorrektur muss vor jeder Messung korrekt durchgeführt werden und passiert folgendermaßen:

Die eigentliche Mittelwertskorrektur erfolgt mittels Differenzverstärker 11, dem ein bestimmter Mittelwert $\overline{PG}$ als Startwert vorgegeben ist. Der Differenzverstärker 11 erzeugt das PG-Signal indem das vom Umgebungslicht bereinigte Lichtsignal vom vorgegebenen Mittelwert $\overline{PG}$ subtrahiert wird. Durch den Differenzverstärker 11 wird das PG-Signal nicht nur vom Mittelwert $\overline{PG}$ bereinigt, sondern auch invertiert und verstärkt. Das PG-Signal wird nun einer Komparatorschaltung zugeführt. Die Komparatorschaltung besteht aus einem oberen Komparator 42, einem unterem Komparator 43 sowie einem Spannungsteiler mit den Widerständen 44, 45 und 46, der die Schwellenwerte bestimmt. Liegt das vom Differenzverstärker 11 erzeugte PG-Signal nun über dem Schwellenwert des oberen Komparators 42, so bedeutet dies, dass der voreingestellte Mittelwert **PG** zu groß gewählt wurde. Dies wird der Einheit PG Control 47 mitgeteilt und diese verringert den Mittelwert $\overline{PG}$ bis dass das PG-Signal unter dem Schwellenwert des oberen Komparators 42 zu liegen kommt. Umgekehrt gilt - wenn das vom Differenzverstärker 11 erzeugte PG-Signal unter dem Schwellenwert des unteren Komparators 43 liegt, so ist der voreingestellte Mittelwert zu klein. Die Einheit PG Control 47 erhöht dann den Mittelwert **PG** bis dass das PG-Signal über dem Schwellenwert des unteren Komparators 43 zu liegen kommt.

In der vorliegenden Regelschaltung befindet sich auch ein Peak Detektor 48, der die maximale Amplitude des PG-Signals bestimmt, welche in bekannter Weise dann erreicht wird, wenn der Druck BP in der Manschette 6 in etwa dem mittleren Blutdruck entspricht. So kann man auch den Peak Detektor 48 dafür verwenden, um den mittleren Blutdruck zu finden. Dabei wird der Druck BP in der Manschette 6 so lange verändert, bis die maximale Amplitude des PG-Signals auftritt. Die gefundene maximale Amplitude des PG-Signals wird nun überprüft, da diese auch von der Extremität E des jeweiligen Patienten abhängt. Ist die Amplitude zu klein, dann wird die Einheit LED Control 33 veranlasst, den Strom und somit die Leuchtstärke der LED 8 zu erhöhen. Umgekehrt wird bei einer zu großen maximalen Amplitude des PG-Signals die Einheit LED Control 33 veranlasst, den Strom durch die LED 8 zu verringern.

Wenn nun der Manschettendruck BP gefunden ist, bei dem die Amplitude des PG-Signals ein Maximum erreicht, kann - wie oben beschrieben - auch die optimale Stromstärke für die LED 8 gefunden werden und mit Hilfe der weiter oben beschriebenen Komparatorschaltung 42-47 das PG-Signal auch optimal von seinem störenden Mittelwert **PG** bereinigt werden. Der gefundene Druck BP entspricht auch dem optimalen Startwert des Arbeitspunktes SP, denn die Variation des Druckes BP wird vorzugsweise durch Veränderung des SP erzielt, wobei die Regelverstärkungen P, I und D auf Null gesetzt werden.

Schließlich ist erfindungsgemäß eine Einrichtung zur Berechnung eines Startwertes für das Arbeitspunktsignal bzw. den Arbeitspunkt SP vorgesehen.

Für die Ermittlung des optimalen Arbeitspunktes SP wird vorzugsweise folgendes Verfahren angewendet: Nachdem

1. durch Variation des SP der Druck BP in der Manschette 6 so verändert wurde, dass der Peak Detektor 48 die maximale Amplitude des PG-Signals ermittelt hat, sowie

2. die optimale Stromstärke für die LED 8 gefunden wurde und

3. das PG-Signal auch optimal von seinem störenden Mittelwert **PG** bereinigt wurde,

berechnet die Verstärkungskontrolle 17 (Fig. 2) aus der maximalen Amplitude des PG-Signals P, I und D und schließt somit den Regelkreis 2. Der Druck in der Manschette 6 beginnt zu pulsieren und gemäß den geforderten Regelbedingung wird das PG-Signal konstant gehalten. Nun wird wieder der SP variiert. Der Systolen - Diastolendetektor 15 trennt die jeweiligen Herzzyklen und bringt sie in Bezug zum jeweiligen dazugehörigen SP, damit sie für die Bestimmung des optimalen SP zu einer Beurteilung herangezogen werden können. Es wird vorzugsweise ein typischer Herzzyklus für jeden unterschiedlichen SP zur Beurteilung herangezogen. Folgende Beurteilungskriterien können angewendet werden: Die Amplitude von BP, das Verhältnis von mittleren Druck zur Druckamplitude, das Verhältnis von mittleren Druck zum diastolischen Druck, Druckanstieg und -Abfall, zeitliche Zusammenhänge und viele mehr. Gemäß der Fuzzy-Logik im Regelkreis 19 können nun zum Beispiel folgende Fuzzy Kriterien formuliert werden und die jeweiligen Herzschläge bzw. Herzzyklen beurteilt werden:

- BP Amplitude des jeweiligen Herzzyklus ist im Bereich der maximalen BP Amplitude

- Verhältnis von mittleren Druck zur Druckamplitude liegt im physiologischen Bereich

- Verhältnis von mittleren Druck zum diastolischen Druck liegt im physiologischen Bereich

- Druckanstieg und -abfall liegen im physiologischen Bereich

- zeitliche Zusammenhänge liegen im physiologischen Bereich

- usw.

Gemäß dieser Fuzzy Kriterien können die jeweiligen Herzzyklen, z.B. mit einem einfachen Notensystem, beurteilt werden. Der Herzzyklus mit den besten Noten hat den optimalen SP, bei Gleichstand wird der Mittelwert der besten SP's herangezogen. Somit kann der optimale Startwert des Arbeitspunktes bzw. des Arbeitssignals SP gefunden und allen konzentrischen Regelkreisen mitgeteilt werden. Die eigentliche Messung kann somit beginnen, da alle Anfangswerte von SP, **PG, P,** I, D sowie der optimale LED-Strom des Regelungssystems ermittelt wurden. Diese werden nun von allen konzentrischen Reglern überwacht und ggf. verändert, womit eine langfristige kontinuierliche Messung des Blutdruckes ermöglicht wird.

**Patentansprüche**

1. Verfahren zur Regelung des Druckes in zumindest einer aufblasbaren Manschette, vorzugsweise einer Fingermanschette (6), eines Blutdruckmessgerätes, welches eine plethysmographische Sensoreinrichtung (8, 9) aufweist, wobei ein plethysmographisches Signal PG und ein Manschettendrucksignal BP erfasst werden, wobei

   a) in einem ersten, inneren Regelkreis das Manschettendrucksignal BP als Regelgröße verwendet und als erstes Eingangssignal einem Differenzverstärker (10) zugeführt wird,

   b) in einem zweiten, äußeren Regelkreis das plethysmographische Signal PG um seinen Mittelwert $\overline{PG}$ bereinigt einer Regeleinrichtung, vorzugsweise einem PID-Regler, zugeführt zu einem Arbeitspunktsignal SP addiert und ein Sollwertsignal SW generiert wird, welches als zweites Eingangssignal dem Differenzverstärker zugeführt wird, und

   c) mit dem Ausgangssignal AS des Differenzverstärkers zumindest ein mit einer Druckquelle (4) verbundenes Ventil, vorzugsweise ein Proportionalventil (3, 25, 27) zur Regelung des Druckes in der Manschette (6) angesteuert wird.

2. Verfahren nach Anspruch 1, **dadurch gekennzeichnet, dass** in einem dritten Regelkreis der Mittelwert $\overline{PG}$ aus dem plethysmographischen Signal PG bestimmt und als Eingangsgröße des zweiten Regelkreises laufend korrigiert wird.

3. Verfahren nach Anspruch 1 oder 2, **dadurch gekennzeichnet, dass** in einem vierten Regelkreis die Verstärkungsparameter P, I und/oder D anhand des plethysmographischen Signals PG und des Manschettendrucksignal BP optimiert und als Eingangsgrößen des PID-Reglers laufend korrigiert werden.

4. Verfahren nach einem der Ansprüche 1 bis 3, **dadurch gekennzeichnet, dass** in einem fünften Regelkreis das Arbeitspunktsignal SP in Abhängigkeit des Integrals des plethysmographischen Signals PG nachgeregelt wird.

5. Verfahren nach einem der Ansprüche 1 bis 4, **dadurch gekennzeichnet, dass** in einem sechsten Regelkreis das Arbeitspunktsignal SP auf der Basis abgeleiteter Größen, wie z.B. Amplitude, Mittelwert, Signalform etc., des plethysmographischen Signals PG und des Manschettendrucksignals BP mit Hilfe eines Fuzzy-Logik-Ansatzes nachgeregelt wird.

6. Verfahren nach einem der Ansprüche 1 bis 5, **dadurch gekennzeichnet, dass** in einem siebenten Regelkreis das Arbeitspunktsignal SP in Abhängigkeit der Pulswellenform des Manschettendrucksignals BP nachgeregelt wird.

7. Verfahren nach einem der Ansprüche 1 bis 6, **dadurch gekennzeichnet, dass** in einem achten Regelkreis das Arbeitspunktsignal SP mit Hilfe neuronaler Netze, autoregressiver Modelle oder selbstlernender Modelle nachgeregelt wird.

8. Verfahren nach einem der Ansprüche 1 bis 7, **dadurch gekennzeichnet, dass** das Manschettendrucksignal BP einem Systolen-/Diastolendetektor zugeführt wird, dessen Ausgangssignal als Regelgröße in zumindest einem der Regelkreise drei bis acht verwendet wird.

9. Vorrichtung zur Regelung des Druckes in zumindest einer aufblasbaren Manschette, vorzugsweise einer Fingermanschette (6), eines Blutdruckmessgerätes, welches eine plethysmographische Sensoreinrichtung (8, 9) zur Erfassung eines plethysmographischen Signals PG und einen Drucksensor (7) zur Erfassung eines Manschettendrucksignals BP aufweist, wobei zwei auf einen Differenzverstärker (10) wirkende Regelkreise (1, 2) vorgesehen sind, wobei der erste, innere Regelkreis (1) das Manschettendrucksignals BP als erste Regelgröße verwendet und der zweite, äußere Regelkreis (2) eine Regeleinrichtung (12), vorzugsweise einen PID-Regler, aufweist, welche bzw. welcher aus dem plethysmographischen Signal PG einen Sollwert SW als zweite Regelgröße generiert, und der Differenzverstärker (10) ausgangsseitig zumindest ein mit einer Druckquelle (4) verbundenes Ventil, vorzugsweise ein Proportionalventil (3; 25, 27), zur Regelung des Druckes in der Manschette (6) steuert.

10. Vorrichtung nach Anspruch 9, **dadurch gekennzeichnet, dass** der zweite Regelkreis (2) einen Differenzverstärker (11) aufweist, welcher das plethysmographische Signal PG von seinem Mittelwert $\overline{PG}$ subtrahiert, sowie eine Summationseinheit (13), welche ein Arbeitspunktsignal SP addiert.

11. Vorrichtung nach Anspruch 9 oder 10, **dadurch gekennzeichnet, dass** der Differenzverstärker (10) ausgangsseitig über eine nicht-invertierende Verstärkereinheit (23) ein mit der Druckquelle (4) verbundenes Einlassventil (25) und über eine invertierende Verstärkereinheit (24) ein Auslassventil (27) steuert, welche vorzugsweise als Proportionalventile ausgeführt sind und mit der aufblasbaren Manschette (6) in Druckverbindung stehen.

12. Vorrichtung nach einem der Ansprüche 9 bis 11, **dadurch gekennzeichnet, dass** der Differenzverstärker (10) als Komparator ausgeführt ist, welcher zumindest ein digitales Schaltventil zur Regelung des Druckes in der Manschette (6) steuert.

13. Vorrichtung nach einem der Ansprüche 9 bis 12, **dadurch gekennzeichnet, dass** die plethysmographische Sensoreinrichtung (8, 9) eine Einrichtung (28, 40, 41) zur Bereinigung des plethysmographischen Signals PG von Fehllicht, insbesondere von Umgebungslicht, aufweist.

14. Vorrichtung nach einem der Ansprüche 9 bis 13, **dadurch gekennzeichnet, dass** die Lichtquelle (8) der plethysmographischen Sensoreinrichtung (8, 9) eine Einrichtung (33 bis 38) zur Steuerung der Spannung bzw. des Stroms aufweist.

15. Vorrichtung nach einem der Ansprüche 10 bis 14, **dadurch gekennzeichnet, dass** eine Einrichtung (42 bis 47) zur Berechnung eines Startwertes für den Mittelwert **PG** des plethysmographischen Signals vorgesehen ist.

16. Vorrichtung nach einem der Ansprüche 10 bis 15, **dadurch gekennzeichnet**, eine Einrichtung zur Berechnung eines Startwertes für das Arbeitspunktsignal SP vorgesehen ist.

**Claims**

1. Method for controlling the pressure in at least one inflatable cuff, preferably a finger cuff (6), of a blood pressure measuring apparatus with a plethysmographic sensing device (8, 9), whereby a plethysmographic signal PG and a cuff pressure signal BP are obtained, wherein

   a) in a first, inner control loop the cuff pressure signal BP is used as control variable and is fed into a difference amplifier (10) as a first input signal,
   b) in a second, outer control loop the plethysmographic signal PG, with its mean value **PG** suppressed, is fed into a controller, preferably a PID-controller, and is added to a set-point signal SP, and a target signal SW is generated, which is fed into said difference amplifier as a second input signal, and
   c) the output signal AS of the difference amplifier is used to control at least one valve connected to a pressure source (4), i.e. preferably a proportional valve (3; 25, 27), which in turn regulates the pressure in the cuff (6).

2. Method according to claim 1, **characterized in that** the mean value **PG** of the plethysmographic signal PG is determined in third control loop and continuously corrected as input signal of the second control loop.

3. Method according to claim 1 or 2, **characterized in that** the amplification parameters P, I and/or D are optimised in a fourth control loop by means of the plethysmographic signal PG and the cuff pressure signal BP, and are continuously corrected as inputs to the PID-controller.

4. Method according to any of claims 1 to 3, **characterized in that** in a fifth control loop the set-point signal SP is readjusted, depending on the integral of the plethysmographic signal PG.

5. Method according to any of claims 1 to 4, **characterized in that** in a sixth control loop the set-point signal SP is readjusted on the basis of derived quantities, such as amplitude, mean value, wave form etc., of the plethysmo-graphic signal PG and the cuff pressure signal BP, using a fuzzy-logic-approach.

6. Method according to any of claims 1 to 5, **characterized in that** in a seventh control loop the set-point signal SP is readjusted in dependence of the pulse waveform of the cuff pressure signal BP.

7. Method according to any of claims 1 to 6, **characterized in that** in an eighth control loop the set-point signal SP is readjusted by means of neural networks, autoregressive models or self-learning models.

8. Method according to any of claims 1 to 7, **characterized in that** the cuff pressure signal BP is fed to a systole/diastole detector whose output signal is used as control variable in at least one of control loops three to eight.

9. A device for controlling the pressure in at least one inflatable cuff, preferably a finger cuff (6), of a blood pressure measuring apparatus, which has a plethysmo-graphic sensor device (8, 9) for obtaining a plethysmo-graphic signal PG and a, pressure sensor (7) for obtaining a cuff pressure signal BP, wherein two control loops (1, 2) acting on a difference amplifier (10) are provided, where the first, inner control loop (1) uses the cuff pressure signal BP as a first control variable and where the second, outer control loop (2) is provided with a controller (12), preferably a PID-controller, which generates a target variable SW from the plethysmographic signal PG as a second control variable, and where the output of the difference amplifier (10) controls at least one valve connected to a pressure source (4), i.e. preferably a proportional valve (3; 25, 27), thereby regulating the pressure in the cuff (6).

10. Device according to claim 9, **characterized in that** the second control loop (2) is provided with a difference amplifier (11), which subtracts the plethysmographic signal PG from its mean value **PG**, and with

a summation unit (13) adding a set-point signal SP.

**11.** Device according to claim 9 or 10, **characterized in that** said difference amplifier (10) controls an inlet valve (25) connected to a pressure source (4) via a non-inverting amplifier unit (23) and an outlet valve (27) via an inverting amplifier unit (24), said valves preferably being designed as proportional valves which are pressure-connected to the inflatable cuff (6).

**12.** Device according to any of claims 9 to 11, **characterized in that** said difference amplifier (10) is designed as a comparator which actuates at least one digital switching valve for pressure regulation in the cuff (6).

**13.** Device according to any of claims 9 to 12, **characterized in that** the plethysmographic sensor (8, 9) is furnished with a device (28, 40, 41) for the elimination of stray light, in particular ambient light, from the plethysmo-graphic signal PG.

**14.** Device according to any of claims 9 to 13, **characterized in that** the light source (8) of the plethysmographic sensor (8, 9) is furnished with circuitry (33 to 38) for controlling its voltage or current.

**15.** Device according to any of claims 10 to 14, **characterized in that** a device (42 to 47) is provided for computing an initial value for the mean value **PG** of the plethysmo-graphic signal.

**16.** Device according to any of claims 10 to 15, **characterized in that** a device is provided for computing an initial value for the set-point signal SP.

**Revendications**

**1.** Procédé de régulation de la pression dans au moins un manchon gonflable, de préférence un manchon digital (6) d'un tensiomètre ayant une installation de capteur pléthysmographique (8, 9), pour saisir un signal pléthysmographique PG et un signal de pression de manchon BP, selon lequel :

a) dans un premier circuit de régulation intérieur on utilise le signal de pression BP du manchon comme grandeur de régulation et l'applique comme premier signal d'entrée à un amplificateur de différence (10),
b) dans un second circuit de régulation, extérieur, on élimine du signal pléthysmographique PG, sa valeur moyenne $\overline{P}\,\overline{G}$ que l'on applique à une installation de régulation, de préférence à un régulateur PID pour l'additionner à un signal de point de fonctionnement SP et on génère un signal de valeur de consigne SW appliqué comme second signal d'entré à l'amplificateur de consigne SW appliqué comme second signal d'entrée à l'amplificateur de différence et
c) avec le signal de sortie AS de l'amplificateur de différence on commande au moins une vanne reliée à une source de pression (4), de préférence une vanne proportionnelle (3, 25, 27) pour réguler la pression dans le manchon (6).

**2.** Procédé selon la revendication 1, **caractérisé en ce que** dans un troisième circuit de régulation on détermine la valeur moyenne $\overline{P}\,\overline{G}$ du signal pléthysmographique PG et on corrige cette grandeur en permanence comme grandeur d'entrée du second circuit de régulation.

**3.** Procédé selon la revendication 1 ou 2, **caractérisé en ce que** dans un quatrième circuit de régulation on optimise les paramètres d'amplification P, I et/ou D à l'aide du signal pléthysmographique PG et on optimise le signal de pression de manchon BP et on corrige ce signal en continu comme grandeur d'entrée du régulateur PID.

**4.** Procédé selon l'une des revendications 1 à 3, **caractérisé en ce que** dans un cinquième circuit de régulation on assure l'asservissement du signal de point de fonctionnement SP en fonction de l'intégrale du signal pléthysmographique PG.

**5.** Procédé selon l'une des revendications 1 à 4, **caractérisé en ce que** dans un sixième circuit de régulation on asservit le signal du point de fonctionnement SP sur la base des grandeurs déduites telles que par exemple l'amplitude, la valeur moyenne, la forme du signal, etc..., du signal pléthysmographique PG et du signal de pression de manchon PG à l'aide d'une application de logique floue.

**6.** Procédé selon l'une des revendications 1 à 5, **caractérisé en ce que** dans un septième circuit de régulation on asservit le signal du point de fonctionnement SP en fonction de la forme de l'onde impulsionnelle du signal de pression de manchon BP.

**7.** Procédé selon l'une des revendications 1 à 6, **caractérisé en ce que** dans un huitième circuit de régulation on asservit le signal du point de fonctionnement SP à l'aide de réseaux neuronaux, de modèles autorégressifs ou de modèles intelligents.

**8.** Procédé selon l'une des revendications 1 à 7, **caractérisé en ce qu'** on applique le signal de pression de manchon BP à un détecteur de systoles/diastoles dont le signal de sortie est utilisé comme grandeur de régulation dans au moins l'un des trois à huit circuits de régulation.

**9.** Dispositif de régulation de la pression dans au moins un manchon gonflable, de préférence un manchon digital (6) d'un tensiomètre comportant une installation de capteur pléthysmographique (8, 9) pour saisir un signal pléthysmographique PG et un capteur de pression (7) pour saisir un signal de pression de manchon BP, comprenant deux circuits de régulation (1, 2) agissant sur un amplificateur de différence (10), le premier circuit de régulation (1), interne, utilisant le signal de pression de manchon BP comme première grandeur de régulation et le second circuit de régulation (2), externe, ayant une installation de régulation (12), de préférence un régulateur PID, qui, à partir du signal pléthysmographique PG, génère une valeur de consigne SW comme seconde grandeur de régulation et l'amplificateur de différence (10) commande en sortie au moins une vanne reliée à une source de pression (4), de préférence une vanne proportionnelle (3, 25, 27) pour réguler la pression dans le manchon (6).

**10.** Dispositif selon la revendication 9, **caractérisé en ce que** le second circuit de régulation (2) comporte un amplificateur de différence (11) qui retranche le signal pléthysmographique PG de sa valeur moyenne $\overline{P}\,\overline{G}$ ainsi qu'une unité d'addition (13) qui additionne un signal de point de fonctionnement SP.

**11.** Dispositif selon la revendication 9 ou 10, **caractérisé en ce que** l'amplificateur de différence (10) est relié en sortie par un amplificateur non inversé (23) à la vanne d'admission (25) reliée à la source de pression (4) et par un amplificateur inversé (24) à la vanne de sortie (27) pour les commander, ces vannes étant de préférence des vannes proportionnelles et communiquant en pression avec le manchon gonflable (6).

**12.** Dispositif selon l'une des revendications 9 à 11, **caractérisé en ce que** l'amplificateur de différence (10) est un comparateur qui commande au moins un signal de commutation numérique pour réguler la pression dans le manchon (6).

**13.** Dispositif selon l'une des revendications 9 à 12, **caractérisé en ce que** l'installation de capteur pléthysmographique (8, 9) comprend une installation (28, 40, 41) pour éliminer du signal pléthysmographique PG, la lumière parasite, notamment la lumière ambiante.

**14.** Dispositif selon l'une des revendications 9 à 13, **caractérisé en ce que** la source lumineuse (8) de l'installation de capteur pléthysmographique (8, 9) comporte une installation (33-38) pour commander la tension ou le courant.

**15.** Dispositif selon l'une des revendications 10 à 14, **caractérisé par** une installation (42-47) pour calculer la valeur initiale de la valeur moyenne $\overline{P}\,\overline{G}$ du signal pléthysmographique.

**16.** Dispositif selon l'une des revendications 10 à 15, **caractérisé par** une installation pour calculer la valeur initiale du signal de point de fonctionnement SP.

Fig. 1

Fig. 2

*Fig. 3*

*Fig. 4*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0537383 A1
- US 4510940 A
- US 4539997 A
- US 4406289 A
- WO 0059369 A
- WO 0059369 A2

**In der Beschreibung aufgeführte Nicht-Patentliteratur**

- Methodik und Ergebnisse fortlaufender Blutdruck-schreibung am Menschen. **WAGNER R.** Leipzig. Georg Thieme Verlag, 1942
- **WAGNER R.** Vereinfachtes Verfahren zur fortlaufenden Aufschrift des Blutdruckes beim Menschen. *Zschr. Biol,* 1960, vol. 112